# EUROPEAN PATENT APPLICATION

(11) **EP 2 050 456 A1**
(43) Date of publication of application: **22.04.2009**
(21) Application number: 07792297.9
(22) Date of filing: 09.08.2007
(51) Int. Cl.: A61K 31/785, A61K 9/20, A61K 9/30, A61K 9/32, A61K 9/36, A61K 9/42, A61K 47/04, A61K 47/10, A61K 47/14, A61K 47/34, A61K 47/38, A61P 3/06, A61P 3/12

(54) **TABLET**

(30) Priority: 09.08.2006 JP 2006216447
(71) Applicant: Mitsubishi Tanabe Pharma Corporation, Chuo-ku Osaka-shi Osaka 541-8505 (JP)
(72) Inventor: SUZUKI, Tetsuya, Chuo-ku, Osaka-shi Osaka 541-8505 (JP); TOKUTOMI, Koji, Chuo-ku, Osaka-shi Osaka 541-8505 (JP); NOMURA, Tatsuo, Chuo-ku, Osaka-shi Osaka 541-8505 (JP); OHNO, Chika, Chuo-ku, Osaka-shi Osaka 541-8505 (JP); YOSHINARI, Tetsuro, Chuo-ku, Osaka-shi Osaka 541-8505 (JP)
(74) Representative: Merkle, Gebhard
(86) International application number: PCT/JP2007/065647
(87) International publication number: WO 2008/018556

(57) **Abstract**

The present invention provides a novel tablet with improved tablet appearance and improved swallowability. The tablet contains a pharmaceutically acceptable anion exchange resin represented by colestimide as an active ingredient, and has a visibility-resolved tablet edge.

## Description

### Technical Field

The present invention relates to a novel tablet, and more particularly relates to a tablet improved in the appearance of the tablet and swallowability.

### Background Art

Since the dosage of tablets containing an anion exchange resin as an active ingredient is high, the size of the tablets becomes large. Considering the swallowability, Capsule/Oval-shaped tablets are considered to be preferable. However, Capsule/Oval-shaped tablets, unlike round tablets, cause distortion in a part of a punch during tableting, which leads to an easy breakage of the punch. The conventionally-employed methods of tableting while maintaining a given amount of water (patent reference 1 and patent reference 2) require a greater compression pressure, and therefore, are insufficient as a production method of a core tablet.

On the other hand, as regards a tablet coating containing an anion exchange resin as an active ingredient, a method using a colestyramine resin, which includes heat-dissolving stearic acid in polyethylene glycol for coating without using a solvent is known (patent reference 2). However, the tablet coated by this method is inferior in the storage stability in an open state since the tablet is disintegrated by moisture adsorption in several hours at room temperature, and the stability after opening the package is extremely poor. Furthermore, since the coating film has a low strength and is easily friable, the tablets have a defect in that they are broken in the packaging process or during transportation.

In addition, a method including coating an anion exchange resin with hydroxypropylcellulose is also known (patent reference 3). However, the tablets coated by this method are defective in that they adhere to each other to impair flowability since hydroxypropylcellulose used for a coating film absorbs moisture and increases its viscosity, even though the stability under a humidified environment is improved.

To improve them, a core tablet further improved in the tableting compressibility by using an anion exchange resin as an active ingredient and containing a given amount of water and, as a secondary component, silicon dioxide conventionally used as a fluidizing agent has been produced (patent reference 4). Furthermore, an easily swallowable oral cholesterol lowering agent, which is stable and does not lose fluidity easily even in a humidified environment, has been found by using hydroxypropylmethylcellulose having a higher viscosity than that of hydroxypropylmethylcellulose generally used for a coating (patent reference 4).

As mentioned above, although various improving techniques have been found for increasing the size of tablets, the tablet size may be further increased when the dose is changed for addition of efficacy and the like. Accordingly, a tablet superior in the tableting compressibility and swallowability, which is stable and does not lose flowability easily even in a humidified environment, is desired.
patent reference 1: JP-A-2-286621
patent reference 2: JP-A-3-236326
patent reference 3: JP-A-6-157325
patent reference 4: JP-A-7-97330

### Disclosure of the Invention

### Problems to be Solved by the Invention

The problem of the present invention is provision of a novel tablet containing an anion exchange resin as an active ingredient, and more particularly, provision of a novel tablet having improved tablet appearance and swallowability.

### Means of Solving the Problems

The present inventors have conducted intensive studies in view of the above-mentioned problems and found that the tablet appearance and swallowability of a tablet containing an anion exchange resin as an active ingredient can be markedly improved by changing the edge part thereof to a invisible edge, which resulted in the completion of the present invention.

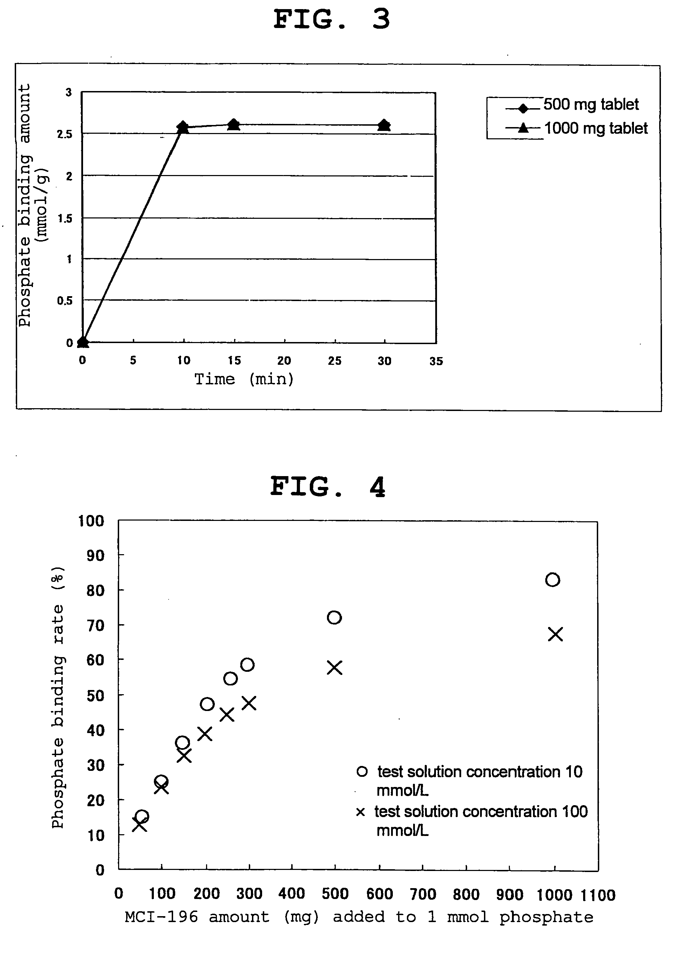

Accordingly, the gist of the present invention is as follows.
(1) A tablet comprising a pharmacologically acceptable anion exchange resin as an active ingredient, which has a visibility-resolved tablet edge.
(2) The tablet of the above, wherein the pharmaceutically acceptable anion exchange resin has bile acid binding capacity.
(3) The tablet of the above, wherein the pharmaceutically acceptable anion exchange resin is selected from colestimide, colestyramine resin, colestipol, sevelamer and colesevelam.
(4) The tablet of the above, wherein the pharmaceutically acceptable anion exchange resin is synthesized by a polymerization reaction of an epichlorohydrin derivative and amine.
(5) The tablet of the above, wherein the pharmaceutically acceptable anion exchange resin is colestimide.
(6) The tablet of the above, comprising a coated core tablet comprising a pharmaceutically acceptable anion exchange resin.
(7) The tablet of the above, wherein the coating is formed by a coating agent containing hydroxypropylmethylcellulose and propylene glycol, polyethylene glycol or glycerol esters of fatty acids.
(8) The tablet of the above, wherein the coating is formed by a coating agent comprising hydroxypropylmethylcellulose and glycerol esters of fatty acids.
(9) The tablet of the above, wherein the core tablet comprises a fluidizing agent.
(10) The tablet of the above, wherein the fluidizing agent is silicon dioxide or light anhydrous silicic acid.
(11) A tablet comprising a core tablet comprising colestimide as an active ingredient and light anhydrous silicic acid, which is coated with a coating agent comprising hydroxypropylmethylcellulose and glycerol esters of fatty acids.
(12) The tablet of the above, which is a 1000 mg tablet.
(13) The tablet of the above, which shows a phosphate binding behavior shown in Fig. 3.
(14) A test method for measuring phophate binding behavior of one tablet comprising colestimide as an active ingredient according to the Paddle method (50 rpm), which comprises using a test solution having a pH of 6.5 to 8.0, and consisting of a phosphate buffer having a phosphate concentration of 10 mmol/L to 100 mmol/L.
(15) The test method of the above, wherein the test solution consists of 10 mmol/L phosphate buffer (900 ml, pH 7).
(16) The test method of the above, which is performed in the absence of a competitive ion.

### Effect of the Invention

According to the present invention, a novel tablet preparation comprising an anion exchange resin as an active ingredient, which is improved in the tablet appearance and swallowability, can be provided.

### Brief Description of the Drawings

Fig. 1 is a photograph showing the shape of the tablet obtained in Example 1.
Fig. 2 is a photograph showing the shape of the tablet obtained in Example 2.
Fig. 3 shows the results of a phosphate binding test.
Fig. 4 shows the relationship between phosphate concentration and the amount of colestimide (MCI-196) added.
Fig. 5 shows the linearity of the amount of colestimide (MCI-196) added and phosphate binding amount in 10 mmol/L phosphate buffer (50 mL).
Fig. 6 shows the linearity of the amount of colestimide (MCI-196) added and phosphate binding amount in 50 mmol/L phosphate buffer (50 mL).
Fig. 7 shows the linearity of the amount of colestimide (MCI-196) added and phosphate binding amount in 100 mmol/L phosphate buffer (50 mL).
Fig. 8 shows phosphate exchange capacity in the presence of sodium chloride.
Fig. 9 shows pH dependency of the phosphate exchange capacity.

### Best Mode for Carrying out the Invention

In the present invention, the pharmaceutically acceptable anion exchange resin means an anion exchange resin that can be administered as a pharmaceutical product, preferably an anion exchange resin having bile acid binding capacity.

One most preferable example thereof is colestimide (2-methylimidazole-epichlorohydrin copolymer). Colestimide has an irregularly complex steric structure, is shown by the basic structure of the following formula (I), and has a partial structure shown by the following formula (II). It is obtained by a polymerization reaction of an epichlorohydrin derivative and an amine represented by an imidazole derivative, i.e., the production method described in JP-A-60-209523.

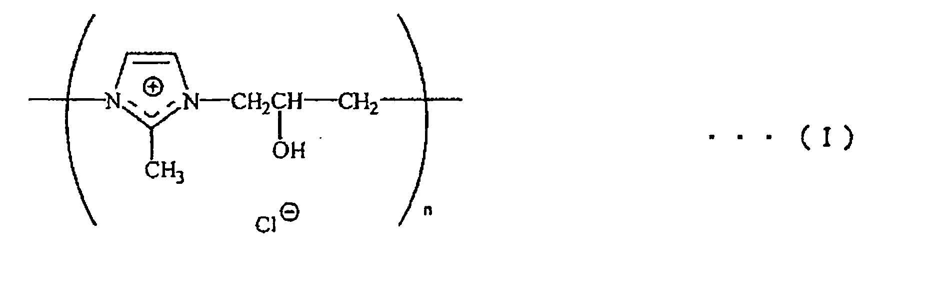

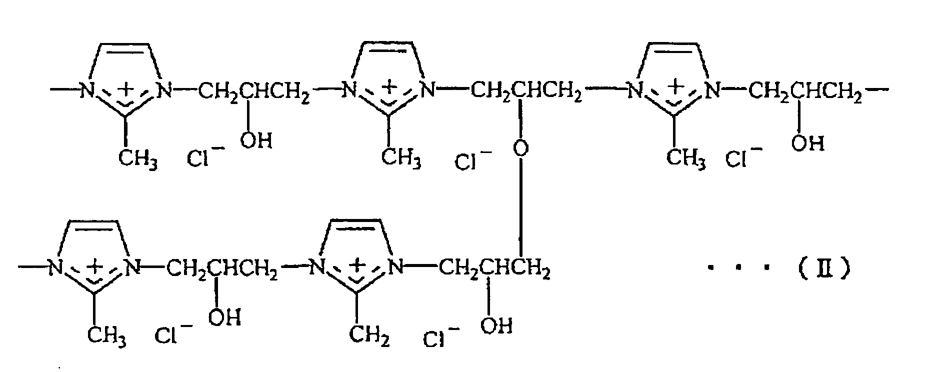

While colestimide is registered by a general name of colestimide (chemical name: 2-methylimidazole-epichlorohydrin copolymer) in JAN, it is registered by a general name of colestilan (chemical name: 2-methylimidazole polymer with 1-chloro-2,3-epoxypropane) in INN.

Examples of other preferable anion exchange resins include the aforementioned colestyramine resin, colestipol (N-(2-aminoethyl)-N'-[2-[(2-amino-ethyl)amino]ethyl]-1,2-ethanediamine polymer added with (chloromethyl)oxirane) and the like, which are commercially available from Sigma Ltd. Colestyramine resin is a strong basic anion exchange resin containing a styrene-divinylbenzene copolymer added with a quaternary ammonium group, whose basic structure is represented by the following formula (III).

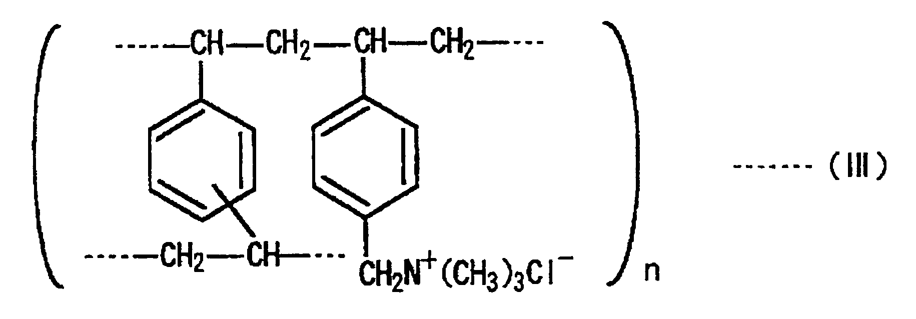

In addition, the basic structure of sevelamer is represented by the following formula, and the sevelamer can be produced according to the method described in US Patent No. 5496545 or a method analogous thereto. A salt thereof is not limited to hydrochloride.

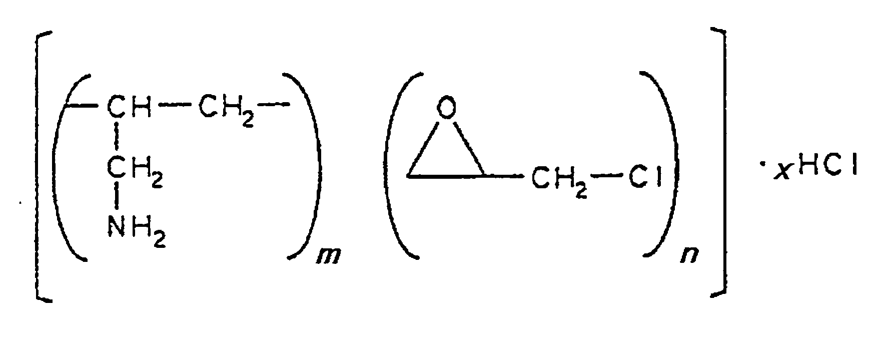

The basic structure of colesevelam is represented by the following formula, and colesevelam can be produced according to the method described in US Patent No. 5607669 or a method analogous thereto. A salt thereof is not limited to hydrochloride and may be carbonate.

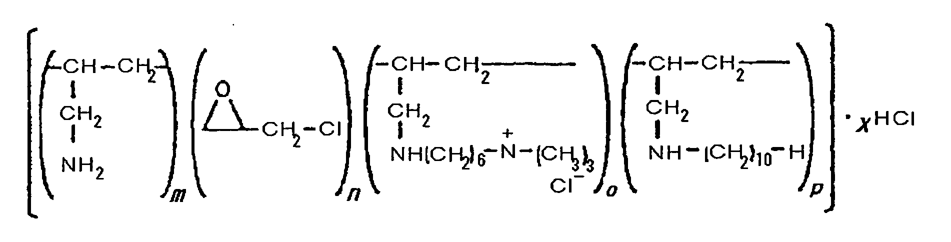

Other anion exchange resins described in each of National Publication of International Patent Application Nos. H9-504782, 9-500368, 10-501264, 10-501842, 11-507093, 11-512074 and 5-512332, and JP-A-8-208750, 9-202732, 10-114661 and 11-228449 and the like can also be used in the present invention as long as they do not go beyond the gist of the present invention.

Examples of the epichlorohydrin derivative include, besides epichlorohydrin, 2-methylepichlorohydrin, 2-ethylepichlorohydrin and the like.

Examples of the amines include amines represented by imidazole derivatives.

In the tablet of the present invention, water is mixed with the above-mentioned anion exchange resin to contain 14 - 20 wt%, preferably 15 - 19 wt%, of water. In this case, a binding solution such as hydroxypropylcellulose (hereinafter sometimes to be referred to as HPC) and the like may be added besides water. Where necessary, moreover, a fluidizing agent is added to the anion exchange resin. The content of the fluidizing agent to be added is not more than 2 wt%, preferably 0.2 - 1.0 wt%. Examples of the fluidizing agent to be used in the present invention include hydrated silicon dioxide, light anhydrous silicic acid and the like.

After adding a fluidizing agent as necessary and mixing same as mentioned above, the mixture is granulated by a speed mill. A lubricant such as hydrogenated oil and the like is added thereto and, after mixing, the mixture is tableted. Here, when the above-mentioned water content exceeds 20%, the tablet unpreferably becomes like a sponge. In addition, when a fluidizing agent is to be added, an amount of the fluidizing agent exceeding 2% degrades the tablet compressibility.

A core tablet containing an anion exchange resin thus produced is coated with a coating solution preferably containing hydroxypropylmethylcellulose of 10 - 30 cSt (centistokes: defined in the Japanese Pharmacopoeia as the viscosity of 2% aqueous solution at 20°C) (hereinafter hydroxypropylcellulose is sometimes referred to as HPMC, and particularly, HPMC having the aforementioned viscosity is sometimes referred to as highly viscous HPMC) by a suitable coating apparatus. In a preferable embodiment of the present invention, the coating solution here is added with, in addition to HPMC, propylene glycol, polyethylene glycol or glycerol esters of fatty acids. By adding these to the coating solution, a tablet with a visibility-resolved edge can be obtained, as shown in the below-mentioned Examples. A more effective combination of coating agents for forming a visibility-resolved edge is that of HPMC and glycerol esters of fatty acids.

In the present invention, water can be used as a solvent of a coating solution. In coating using a water solvent, a core tablet having a water content of less than 14% relative to an anion exchange resin becomes like a sponge by swelling during coating to cause breakage of a coating film. Accordingly, for coating using a water solvent, a core tablet needs to contain water of not less than 14%, as mentioned above.

In the present invention, moreover, solid components such as titanium oxide, talc, low-substituted HPC, ethylcellulose, dye and the like can be added to a coating solution for any purpose. In this case, when the amount of these solid components is set to not more than 50 wt% relative to HPMC, the strength of a coating film can be increased. When the amount of the solid component exceeds 50%, the stability of the coating tablet to humidity decreases markedly.

As a coating solution, HPMC having low viscosity, pH non-dependent and water-soluble HPC, celluloses such as methylcellulose and the like, polysorbate (polyoxyethylenesorbitan oleic acid ester) and the like can be used alone or in an appropriate combination, besides the above-mentioned highly viscous HPMC, propylene glycol, polyethylene glycol and glycerol esters of fatty acids.

Furthermore, to adjust tablet disintegration time and improve moisture-proof effect of tablets, water-insoluble ethylcellulose and a small amount of wax can be added to these water-soluble celluloses.

When HPC, low viscosity HPMC and the like are used in combination with highly viscous HPMC, the stability of a coating film decreases greatly by mixing them. In this case, therefore, a coating solution comprising HPC, low viscosity HPMC and the like is first applied to a core tablet as an undercoating, and a coating solution comprising highly viscous HPMC, propylene glycol, polyethylene glycol or glycerol esters of fatty acids is applied thereon as an overcoating, whereby a tablet stable even under a humidified environment can be obtained.

When a coating solution comprising highly viscous HPMC, propylene glycol, polyethylene glycol or glycerol esters of fatty acids is used alone, the amount to be coated is preferably 1 - 5 wt% relative to a core tablet containing an anion exchange resin. The tablet thus obtained in the present invention has a coating layer with a thickness of about 30 µm - about 160 µm, preferably about 60 µm- about 120 µm. For double coating, the undercoating is preferably 1 - 4 wt%, and the overcoating is preferably 0.5 - 2 wt%.

The tablet of the present invention obtained above is a tablet preparation having a visibility-resolved edge because the edge of the tablet is invisible. Accordingly, even when the dosage is high and the weight of the whole preparation has increased, the tablet is superior since it has an improved preparation appearance and improved swallowability. In addition, the tablet of the present invention can be changed to have a high dosage form, and is superior in the phophate binding capacity after such change in the dosage form.

While the tablet of the present invention is not particularly limited, a 1000 mg tablet is preferable.

The preparation of the present invention can be used for the prophylaxis or treatment of hypercholesterolemia or hyperphosphatemia.

The dose of the preparation of the present invention can be appropriately determined depending on the active ingredient to be used, the age, health condition and body weight of the patient, severity of disease, the kind and frequency of therapy · treatment to be applied simultaneously, the nature of a desired effect and the like. Taking colestimide as an example, the daily does for an adult is generally 1 - 60 g of the amount of the active ingredient, which can be administered at one time or in several portions a day.

The pH at which a phosphate binding test is performed in the present invention is, nonlimitatively, pH 6.5 to pH 8.0. It is preferably pH 6.7 to pH 7.9, most preferably pH 7.

The concentration of a phosphate buffer at which a phosphate binding test is performed in the present invention is 10 mmol/L to 100 mmol/L, preferably 10 mmol/L.

In the present invention, the phosphate binding test is preferably performed under the condition free of a competitive ion. Examples of the competitive ion include various anions. Specifically, chloride ion, sulfate ion, nitrate ion, acetate ion and the like can be mentioned.
In the present invention, one tablet means, for example, one 1000 mg tablet.

In the present invention, the phosphate buffer preferably means, nonlimitatively, a mixed solution of sodium dihydrogen phosphate (NaH₂PO₄) and disodium hydrogen phosphate (Na₂HPO₄) at a 1:1 ratio.

In the present invention, the Paddle method is, for example, the method described in the Japanese Pharmacopoeia, 15th Edition <6.01> or U.S. Pharmacopoeia 30 <711>.

In the present invention, the phosphate concentration of the test solution can be measured by, for example, liquid chromatography.

### Examples

The present invention is explained in more detail in the following by referring to Examples. However, the invention is not limited thereto as long as the gist of the invention is not deviated. Colestimide (hereinafter sometimes to be referred to as "MCI-196") used below was produced according to the method described in JP-A-60-209523.

### (Example 1)

Colestimide (5313 g, water 5.9%), hydroxypropylcellulose (225 g) and light anhydrous silicic acid (20 g) were added to a high shear granulator (manufactured by POWREX CORPORATION, FM-VG-25) and mixed therein. Purified water (541 g) was added thereto, and the mixture was granulated. The granules were taken out and milled in a milling machine(manufactured by Okada Seiko Co., Ltd., ND-10). Hydrogenated castor oil (3.4 g) was added to the milled granules (1000 g), and the mixture was mixed and tableted to give a core tablet. The size of the core tablet was long diameter 20.2±0.1 mm, short diameter 10.7±0.1 mm and thickness 8.4±0.15 mm.

The obtained core tablet contained 1000 mg of colestimide per tablet. The core tablet was coated under the conditions of inlet temperature 70°C, high-speed spray rate 6 g/min using Hicoater HCT-30 (manufactured by Freund Corporation), whereby the tablet of the present invention was produced. The coating solution had the following composition, and was obtained by dissolving HPMC in water, adding glycerol esters of fatty acids and polysorbate 80, mixing them well and passing the mixture through a 80 mesh sieve, after which used for the coating.

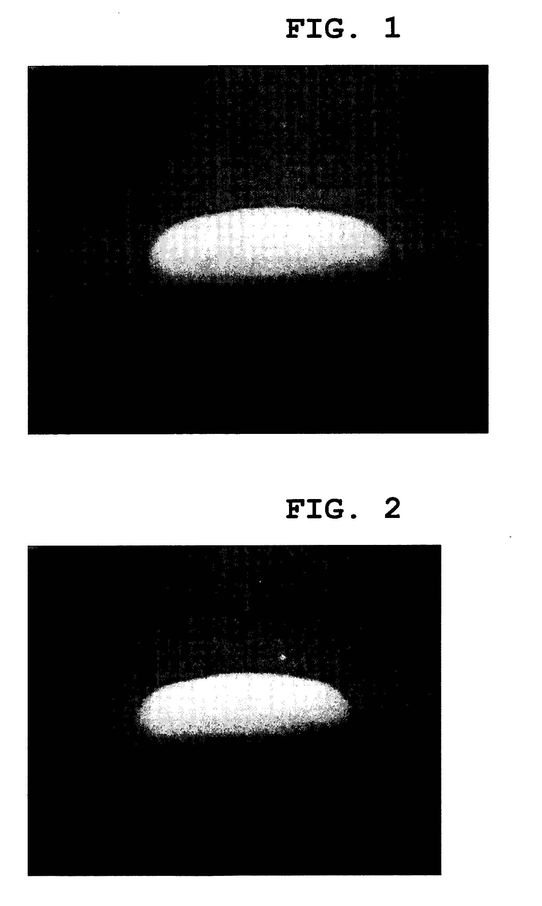

The edge of the obtained coating tablet was evaluated for resolution of edge visibility by visual observation. As a result, the edge visibility was resolved and the tablet had a invisible edge (see Fig. 1).

**[Table 1]**

| coating solution composition | |
|---|---|
| hydroxypropylmethylcellulose 2910 | 5.8 wt% |
| glycerol esters of fatty acids | 1.2 wt% |
| polysorbate | 0.1 wt% |
| purified water | 92.9 wt% |
| | 100.0 wt% |

### (Example 2)

The core tablet obtained in the same manner as in Example 1 was coated under the conditions of inlet temperature 60°C, high-speed spray rate 6 g/min using Hicoater HCT-LABO (manufactured by Freund Corporation), whereby the tablet of the present invention was produced. The coating solution had the following composition, and was obtained by dissolving HPMC in water, adding propylene glycol and talc, mixing them well and passing the mixture through a 80 mesh sieve, after which used for the coating.

The edge of the obtained coating tablet was evaluated for resolution of edge visibility by visual observation. As a result, the edge visibility was resolved and the tablet had a invisible edge (see Fig. 2).

**[Table 2]**

| coating solution composition | |
|---|---|
| hydroxypropylmethylcellulose 2910 | 5.4 wt% |
| propylene glycol | 1.1 wt% |
| talc | 0.5 wt% |
| purified water | 93.0 wt% |
| | 100.0 wt% |

### (Example 3)

Under the following test conditions, the phosphate binding behavior of 500 mg tablets (Cholebine (registered trade mark) 500 mg tablets, available from Mitsubishi Pharma Corporation) and 1000 mg tablets (tablets obtained in Example 1) was evaluated. As a result, the phosphate binding behavior was not different (see Fig. 3). In other words, according to the present invention, a tablet having improved appearance and improved swallowability can be provided, while maintaining a phosphate binding effect similar to that of already commercially available 500 mg tablets.

test conditions
test solution: 10 mmol/L phosphate buffer 900 mL pH=7
a mixed solution of sodium dihydrogen phosphate and disodium hydrogen phosphate at a 1:1 ratio, with a phosphate concentration of 10 mmol/L
test method: the Japanese Pharmacopoeia, 15th Edition, dissolution test, second method (Paddle Method), 50 rpm, 37°C
test conditions of liquid chromatography
detector: electric conductivity detector
separation column: Ion Pac AS11 (4×250 mm)
guard column: Ion Pac AG11 (4×50 mm)
column temperature: 30°C
mobile phase: 25 mmol/L potassium hydroxide solution flow: 1.0 mL/min

### (Example 4)

### 1) Determination of phosphate concentration and amount of colestimide to be added

It has been confirmed that, in the phosphate concentration of 10 - 100 mmol/L, when the amount of colestimide to be added to 1 mmol of phosphate exceeds 200 mg, the phosphate binding amount is deviated from the linearity. The results are shown in Fig. 4.

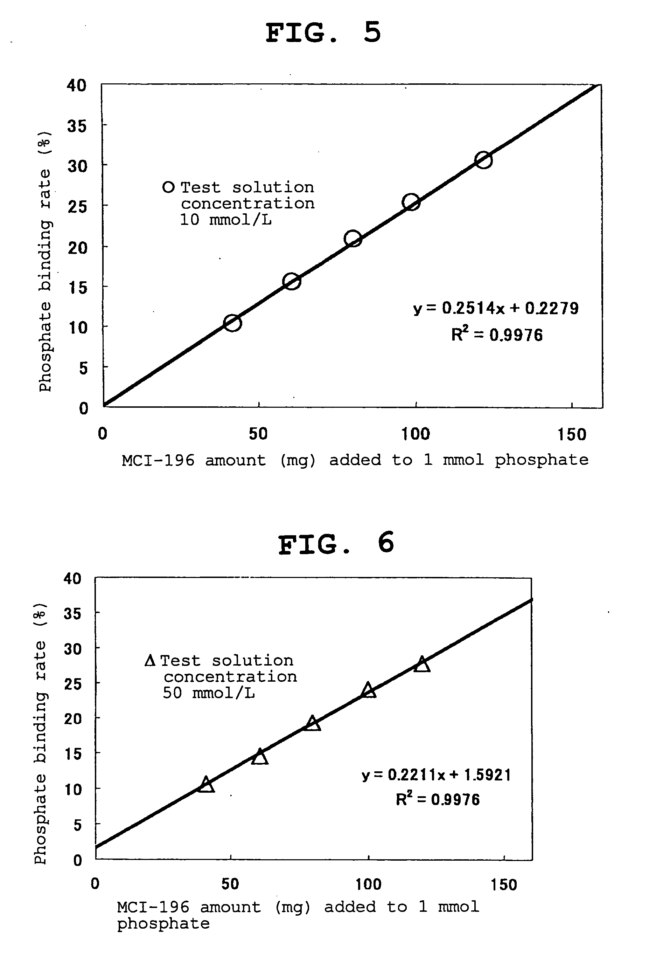

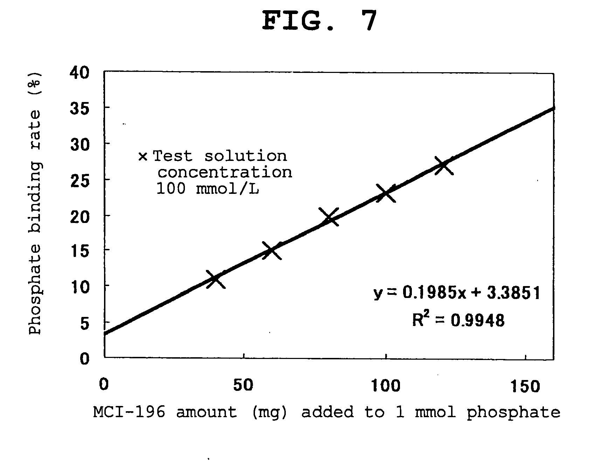

To clarify the optimal phosphate concentration and optimal colestimide amount to be added for a phosphate binding test, a 10 mmol/L phosphate buffer, a 50 mmol/L phosphate buffer and a 100 mmol/L phosphate buffer were respectively examined. The results are shown in Figs. 5 to 7.

From these results, it has been clarified that the optimal amount of colestimide to be added is 40 - 120 mg relative to 1 mmol of phosphate in an binding test, and that the optimal phosphate concentration is 10 mmol/L.

### 2) Consideration relating to phosphate exchange capacity in the presence of sodium chloride

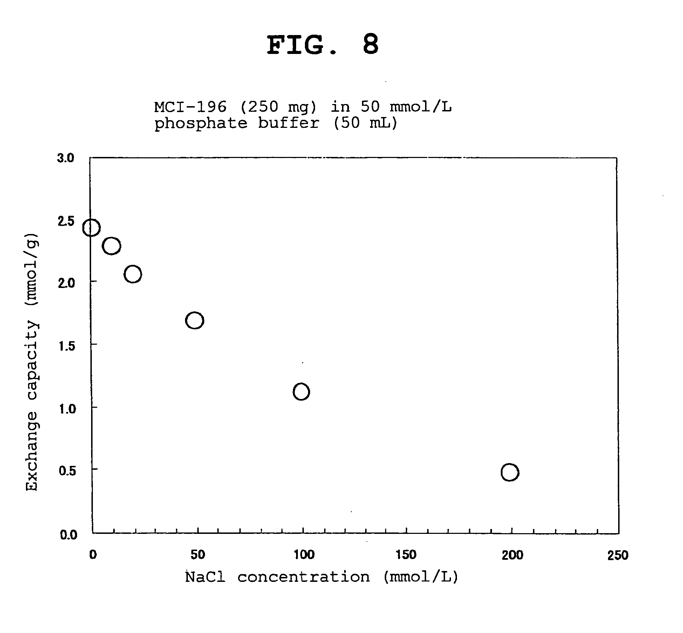

As shown in Fig. 8, it has been confirmed that phosphate exchange capacity decreases when a competitive ion such as sodium chloride is mixed in a test solution.
From the results, it has been clarified that a phosphate binding test should be performed under the condition free of a competitive ion such as sodium chloride in a test solution.

### 3) Consideration relating to pH dependency of phosphate exchange capacity

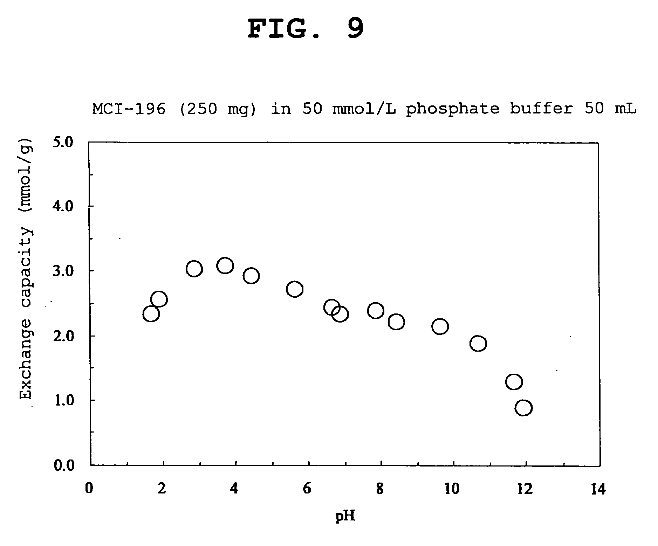

Using a test solution having a phosphate concentration of 10 mmol/L and free of a competitive ion, the pH dependency of phosphate exchange capacity was confirmed.
The results are shown in Fig. 9.
From the results, it has been clarified that a phosphate binding test is preferably performed at around pH 7.

### Industrial Applicability

According to the present invention, a novel tablet preparation can be provided, which contains an anion exchange resin as an active ingredient, and which is improved in the tablet appearance and swallowability.
In addition, according to the test method for measuring the phosphate binding behavior of the present invention, preparations having equivalent quality can be supplied stably. Furthermore, by stably supplying preparations having equivalent quality, a stable therapeutic effect of the preparations can be expected.
The present invention is based on a patent application No. 2006-216447 (filing date: August 9, 2006) filed in Japan, the contents of which are incorporated in full herein by this reference.

## Claims

1. A tablet comprising a pharmacologically acceptable anion exchange resin as an active ingredient, which has a visibility-resolved tablet edge.

2. The tablet of claim 1, wherein the pharmaceutically acceptable anion exchange resin has bile acid binding capacity.

3. The tablet of claim 1 or 2, wherein the pharmaceutically acceptable anion exchange resin is selected from colestimide, colestyramine resin, colestipol, sevelamer and colesevelam.

4. The tablet of claim 1 or 2, wherein the pharmaceutically acceptable anion exchange resin is synthesized by a polymerization reaction of an epichlorohydrin derivative and amine.

5. The tablet of any of claims 1 to 4, wherein the pharmaceutically acceptable anion exchange resin is colestimide.

6. The tablet of any of claims 1 to 5, comprising a coated core tablet comprising a pharmaceutically acceptable anion exchange resin.

7. The tablet of claim 6, wherein the coating is formed by a coating agent containing hydroxypropylmethylcellulose and propylene glycol, polyethylene glycol or glycerol esters of fatty acids.

8. The tablet of claim 6, wherein the coating is formed by a coating agent comprising hydroxypropylmethylcellulose and glycerol esters of fatty acids.

9. The tablet of any of claims 6 to 8, wherein the core tablet comprises a fluidizing agent.

10. The tablet of claim 9, wherein the fluidizing agent is silicon dioxide or light anhydrous silicic acid.

11. A tablet comprising a core tablet comprising colestimide as an active ingredient and light anhydrous silicic acid, which is coated with a coating agent comprising hydroxypropylmethylcellulose and glycerol esters of fatty acids.

12. The tablet of any of claims 1 to 11, which is a 1000 mg tablet.

13. The tablet of any of claims 1 to 12, which shows a phosphate binding behavior shown in Fig. 3.

14. A test method for measuring phophate binding behavior of one tablet comprising colestimide as an active ingredient according to the Paddle method (50 rpm), which comprises using a test solution having a pH of 6.5 to 8.0, and consisting of a phosphate buffer having a phosphate concentration of 10 mmol/L to 100 mmol/L.

15. The test method of claim 14, wherein the test solution consists of 10 mmol/L phosphate buffer (900 ml, pH 7).

16. The test method of claim 14 or 15, which is performed in the absence of a competitive ion.
